# EUROPÄISCHE PATENTSCHRIFT

(11) **EP 2 962 726 B1**
(45) Veröffentlichungstag und Bekanntmachung des Hinweises auf die Patenterteilung: **11.11.2020**
(21) Anmeldenummer: 15001831.5
(22) Anmeldetag: 20.06.2015
(51) Int. Cl.: A61N 1/36, A61F 2/50

(54) **SYSTEM ZUR ANREGUNG VON PHANTOMEMPFINDUNGEN**
SYSTEM FOR EXCITATION OF PHANTOM SENSATIONS
SYSTEME D'EXCITATION DE SENSATIONS FANTOMES

(30) Priorität: 03.07.2014 DE 102014009811
(43) Veröffentlichungstag der Anmeldung: 06.01.2016
(73) Patentinhaber: Meier-Koll, Alfred, 78351 Bodmann-Ludwigshafen (DE)
(72) Erfinder: MEIER-KOLL, Alfred, 78351 Bodmann-Ludwigshafen (DE)
(74) Vertreter: Stadler, Franz

(56) Entgegenhaltungen:
- WO-A1-98/25552
- WO-A1-2013/188650
- DE-A1-102007 013 660
- DE-U1-202011 002 819
- MEIER-KOLL ET AL: "Ein Phantom-Stimulator für amputierte Gliedmaßen", ORTHOPAEDIE-TEC, BUNDESINNUNGSVERBAND FUER ORTHOPAEDIE-TECHNIK, DE, vol. 5, 1 January 2013 (2013-01-01), pages 36-39, XP009186968, ISSN: 0340-5591

## Beschreibung

Die vorliegende Erfindung betrifft ein System zur Anregung von Phantomempfindungen und ein Verfahren zur Herstellung eines Systems zur Anregung von Phantomempfindungen.

Menschen die, beispielsweise als Folge eines Verkehrsunfalles, ein Körperglied, z. B. einen Fuß oder eine Hand, verloren haben können das Körperglied durch eine Prothese ersetzten. Die Prothese ist ein künstliches Bauteil, so dass dadurch der Mensch keine haptischen Wahrnehmungen mehr mit der Prothese aufweist. Dies führt zu eingeschränkten Fähigkeiten des Menschen mit der Prothese, weil aufgrund des nicht vorhandenen Tastsinnes z. B. bei einer Beinprothese in komplizierten Situationen, beispielsweise auf einer Leiter, das Halten des Gleichgewichtes Schwierigkeiten bereitet oder bei einer Handprothese keine haptischen Wahrnehmungen zu einem mit der Handprothese gegriffenen Gegenstand vorhanden sind.

Die DE 600 28 773 T2 zeigt ein System zum gezielten Weitergeben von Stimulationssignalen an einen Patienten mit einem Gliedmaßenstumpf, das Folgendes umfasst: eine Gliedmaßenprothese, die am Gliedmaßenstumpf des Patienten befestigt werden kann, wobei die Gliedmaßenprothese eine Vielzahl von Sensoren aufweist, die sensorische Signale erzeugen; einen Signalgeber zum Erzeugen von Stimulationssignalen, durch die die Stimulation einer oder mehrerer ausgewählter Sinnesnervenfasern eines abgetrennten Nervs eines Gliedes ausgelöst wird; ein Mikroprozessor, der die sensorischen Signale empfängt und so programmiert ist, dass er bewirkt, dass der Signalgeber die Stimulationssignale erzeugt und dass die Stimulationssignale an den einen oder die mehreren ausgewählten Sinnesnervenfasern weitergegeben werden, um dem Patienten Sinnesempfindungen zu vermitteln, die von der Gliedmaßenprothese zu kommen scheinen und die Stimulationssignale elektrisch sind. Das System dient damit zur Behandlung von Phantomgliederschmerzen. Die WO 98/25552 A1 beschreibt Systeme, die einem Prothesenträger sensorisches Feedback über die Umgebung der Prothese zur Verfügung stellen.

Die Aufgabe der vorliegenden Erfindung besteht deshalb darin, ein System zur Anregung von Phantomempfindungen und ein Verfahren zur Herstellung eines Systems zur Anregung von Phantomempfindungen zur Verfügung zu stellen, bei dem die Empfindungen des Menschen bei einem Kontakt der Prothese mit der Umgebung den Empfindungen eines Menschen ohne einer Prothese und ohne amputierten Körperteil ähnlich sind.

Diese Aufgabe wird gelöst mit einem System zur Anregung von Phantomempfindungen von amputierten Körpergliedern, z. B. ein Fuß oder eine Hand, eines Menschen, umfassend: eine Prothese zur Befestigung an dem übrigen, nicht amputierten Körper des Menschen, wenigstens einen in die Prothese eingebauten Sensor zur Erfassung eines Kontaktes zwischen der Prothese und der Umgebung während eines Kontaktzeitraumes, wenigstens eine Reizvorrichtung zur Reizung der Hautoberfläche an dem übrigen, nicht amputierten Körper des Menschen, eine Steuer- und/oder Regeleinheit zur Steuerung und/oder Regelung der Aktivität der wenigstens einen Reizvorrichtung auf der Hautoberfläche, eine Energieversorgungseinheit zur Versorgung der Steuer-und/oder Regeleinheit und der wenigstens einen Reizvorrichtung mit Betriebsenergie, ein Sensorverbindungsmittel zur Übertragung von Sensorsignalen von dem wenigstens einen Sensor zu der Steuer- und/oder Regeleinheit, ein Reizvorrichtungsverbindungsmittel zur Übertragung von Reizsignalen von der Steuer- und/oder Regeleinheit zu der wenigstens einen Reizvorrichtung, wobei die Steuer- und/oder Regeleinheit dahingehend ausgebildet ist, dass während des Kontaktzeitraumes des von dem wenigstens einen Sensor erfassten Kontaktes zwischen der Prothese und der Umgebung und einem Nachzeitraum nach dem Kontaktzeitraum mit der wenigstens einen Reizvorrichtung eine Reizung der Hautoberfläche an dem übrigen, nicht amputierten Körper des Menschen ausführbar ist zur Anregung der Phantomempfindungen, wobei der Nachzeitraum eine Zeitdauer von weniger als 2000 ms beträgt. Die Reizung der Hautoberfläche bewirkt ein Phantomempfinden des Menschen und das Phantomempfinden tritt gleichzeitig mit dem Kontakt zwischen der Prothese und der Umgebung auf, so dass der Mensch mit der Prothese die Umgebung ähnlich ohne dem amputierten Körperglied haptisch wahrnehmen kann.

In einer zusätzlichen Variante ist die Steuer- und/oder Regeleinheit dahingehend ausgebildet, dass außerhalb des Kontaktzeitraumes und des Nachzeitraumes keine Reizung der Hautoberfläche an dem übrigen, nicht amputierten Körper des Menschen ausführbar ist und/oder die Steuer- und/oder Regeleinheit dahingehend ausgebildet ist, dass außerhalb des Kontaktzeitraumes und des Nachzeitraumes eine Aktivität der wenigstens einen Reizvorrichtung ausgeschlossen ist Zweckmäßig ist die wenigstens eine Reizvorrichtung als je eine Elektrode, insbesondere bipolare Elektrode als Elektrodenpaar, und/oder als je eine mechanische bewegliche Reizvorrichtung ausgebildet und/oder das System umfasst mehrere Reizvorrichtungen zum Auflegen auf unterschiedliche Stellen der Hautoberfläche, insbesondere ist mit der Steuer- und/oder Regeleinheit die Aktivität, vorzugsweise der Grad der Aktivität, der mehreren Reizvorrichtungen getrennt steuerbar und/oder regelbar.

In einer ergänzenden Ausführungsform sind in die Prothese mehrere Sensoren zur Erfassung des Kontaktes zwischen der Prothese und der Umgebung an unterschiedlichen Positionen der Prothese eingebaut, so dass der Kontakt der Prothese zu der Umgebung an unterschiedlichen Positionen der Prothese getrennt erfassbar ist und/oder der wenigstens eine Sensor zur Erfassung des Kontaktes zwischen der Prothese und der Umgebung als ein Berührungssensor und/oder ein Drucksensor und/oder als ein induktiver oder kapazitiver Sensor ausgebildet ist. Der Drucksensor erfasst die zwischen der Prothese und der Umgebung beim Kontakt wirkenden Druckkraft, wobei vorzugsweise je größer die Druckkraft ist, desto größer der Grad der Aktivität der dem Drucksensor zugeordnete wenigstens einen Reizvorrichtung steuerbar und/oder regelbar ist und umgekehrt. Die Sensorsignale übermitteln damit auch die von dem Sensor erfasste Druckkraft. Den unterschiedlichen Positionen an der Prothese sind unterschiedliche rezeptive Hautfelder zugeordnet, so dass die Phantomempfindungen getrennt für die unterschiedlichen Positionen der Prothese angeregt werden können.

Zweckmäßig umfasst das System einen Generator zur Aktivierung der wenigstens einen Reizvorrichtung, vorzugsweise ist der Generator in die Steuer- und/oder Regeleinheit eingebaut, und/oder die Energieversorgungseinheit als eine Batterie, insbesondere eine aufladbare Batterie, vorzugsweise Lithium-Ionen-Batterie, ausgebildet ist und/oder das System eine Ladestation zum Aufladen der Batterie umfasst und/oder das Sensorverbindungsmittel als ein drahtloses und/oder als ein drahtgebundenes Sensorverbindungsmittel ausgebildet ist und mit dem Sensorverbindungsmittel Sensordaten von dem wenigstens einen Sensor zu der Steuer- und/oder Regeleinheit und/oder zu dem Generator und/oder umgekehrt übertragbar sind und/oder das Reizvorrichtungsverbindungsmittel als ein drahtloses und/oder als ein drahtgebundenes Reizvorrichtungsverbindungsmittel ausgebildet ist und mit dem Reizvorrichtungsverbindungsmittel Reizsignale, insbesondere elektrischer Strom, von der Steuer- und/oder Regeleinheit und/oder von dem Generator zu der wenigstens einen Reizvorrichtung und/oder umgekehrt übertragbar sind und/oder das Sensorverbindungsmittel und/oder das Reizvorrichtungsverbindungsmittel und/oder die Steuer- und/oder Regeleinheit und/oder der Generator und/oder die wenigstens eine Reizvorrichtung und/oder der wenigstens eine Sensor eine Antenne für Funksignale umfasst. Die wenigstens eine Reizvorrichtung kann auch als eine mechanische Reizvorrichtung mit einem Elektromotor und einer rotierenden Bürste ausgebildet sein, wobei vorzugsweise der Elektromotor mit Funksignalen als Reizsignale steuerbar und/oder regelbar ist und an dem Elektromotor eine Batterie, eine Motorsteuerung und eine Antenne ausgebildet ist.

In einer ergänzenden Ausführungsform sind bei dem System mit mehreren Sensoren an unterschiedlichen Positionen der Prothese und mit mehreren Reizvorrichtungen zum Auflegen auf unterschiedliche Stellen der Hautoberfläche mit der Steuer- und/oder Regeleinheit die mehreren Reizvorrichtungen dahingehend aktivierbar, dass bei einem Kontakt zwischen der Prothese und der Umgebung an je einem Sensor nur wenigstens eine dem je einen Sensor zugeordnete bestimmte Reizvorrichtung aktivierbar ist und wenigstens eine andere, dem je einen Sensor nicht zugeordnete Reizvorrichtung nicht aktivierbar ist. Vorzugsweise sind bei einem simultanen Kontakt zwischen der Prothese und der Umgebung an mehreren Sensoren simultan die diesen Sensoren zugeordneten Reizvorrichtungen simultan aktivierbar.

In einer zusätzlichen Ausführungsform ist mit einem Sensor, insbesondere Drucksensor, ein Parameter für die zwischen einem Abschnitt der Prothese mit dem Sensor und der Umgebung wirkende Druckkraft ermittelbar und in Abhängigkeit von dem Parameter ist der Grad der Aktivität der wenigstens einen Reizvorrichtung steuerbar und/oder regelbar, vorzugsweise je größer der Parameter ist, desto größer der Grad der Aktivität der wenigstens einen Reizvorrichtung ist und umgekehrt, insbesondere ist der Grad der Aktivität der wenigstens einen Reizvorrichtung direkt proportional zu dem Parameter und vorzugsweise ist der Parameter bei einem Drucksensor die zwischen einem Abschnitt der Prothese mit dem Drucksensor und der Umgebung wirkenden Druckkraft selbst.

In einer weiteren Ausführungsform ist die wenigstens eine Reizvorrichtung als je eine Elektrode mit dem Generator bei einer Aktivität der Elektrode mit einem elektrischen Strom als Reizsignal, insbesondere ein elektrischer Strom mit einer in Abhängigkeit von der Zeit veränderlichen Spannung, beispielsweise Sägezahnspannung oder Rechteckspannung, beaufschlagbar, vorzugsweise mit einer Frequenz zwischen 2Hz und 300 Hz, insbesondere zwischen 10 Hz und 150 Hz. Das Reizsignal kann beispielsweise bei einer mechanischen beweglichen Reizvorrichtung auch ein Funksignal sein.

In einer zusätzlichen Ausführungsform ist der Grad der Aktivität der je einen Elektrode umso größer, mit einer je größeren Spannung und/oder einer je größeren Frequenz die je eine Elektrode beaufschlagbar ist und umgekehrt.

In einer zusätzlichen Ausgestaltung ist die wenigstens eine Reizvorrichtung in einen Handschuh und/oder in einen Liner bzw. Strumpf eingebaut und/oder die Prothese ist als eine Beinprothese oder eine Handprothese oder eine Brustprothese ausgebildet, vorzugsweise sind die Sensoren an unterschiedlichen Positionen im Sohlenabschnitt der Beinprothese eingebaut oder die Sensoren sind in unterschiedlichen künstlichen Fingern der Handprothese eingebaut.

In einer zusätzlichen Ausführungsform wird der Generator als ein Bestandteil der Steuer- und/oder Regeleinheit betrachtet, so dass die Steuer- und/oder Regeleinheit den Generator umfasst und vorzugsweise der Generator und die Steuer- und/oder Regeleinheit als gemeinsame oder auch als getrennte Bauteile ausgebildet sind.

In einer weiteren Variante ist mittels eines Betätigungsorganes der Grad der Aktivität der wenigstens einen Reizvorrichtung steuerbar und/oder regelbar und vorzugsweise ist ohne einer Betätigung des Betätigungsorganes der Grad der Aktivität der wenigstens einen Reizvorrichtung bei der aktiven wenigstens einen Reizvorrichtung im Wesentlichen konstant.

Erfindungsgemäßes Verfahren zur Herstellung eines Systems zur Anregung von Phantomempfindungen von amputierten Körpergliedern, z. B. ein Fuß oder eine Hand, eines Menschen, insbesondere eines in dieser Schutzrechtsanmeldung beschriebenen Systems, mit Schritten: zur Verfügung stellen einer Prothese als Komponente des Systems zur Befestigung an dem übrigen, nicht amputierten Körper des Menschen, zur Verfügung stellen von wenigstens einem Sensor als Komponente des Systems zur Erfassung eines Kontaktes zwischen der Prothese und der Umgebung während eines Kontaktzeitraumes, zur Verfügung stellen wenigstens einer Reizvorrichtung als Komponente des Systems zur Reizung der Hautoberfläche an dem übrigen, nicht amputierten Körper des Menschen, zur Verfügung stellen einer Steuer- und/oder Regeleinheit als Komponente des Systems zur Steuerung und/oder Regelung der Aktivität der wenigstens einen Reizvorrichtung auf der Hautoberfläche, zur Verfügung stellen einer Energieversorgungseinheit als Komponente des Systems zur Versorgung der Steuer- und/oder Regeleinheit und der wenigstens einen Reizvorrichtung mit Betriebsenergie, zur Verfügung stellen eines Sensorverbindungsmittels als Komponente des Systems zur Übertragung von Sensorsignalen von dem wenigstens einen Sensor zu der Steuer-und/oder Regeleinheit, zur Verfügung stellen eines Reizvorrichtungsverbindungsmittel als Komponente des Systems zur Übertragung von Reizsignalen von der Steuer- und/oder Regeleinheit zu der wenigstens einen Reizvorrichtung, vorzugsweise zur Verfügung stellen eines Generators als Komponente des Systems zur Aktivierung der wenigstens einen Reizvorrichtung, wobei die Steuer- und/oder Regeleinheit dahingehend zur Verfügung gestellt wird, dass während des Kontaktzeitraumes des von dem wenigstens einen Sensor erfassten Kontaktes zwischen der Prothese und der Umgebung und in einem Nachzeitraum nach dem Kontaktzeitraum mit der wenigstens einen Reizvorrichtung eine Reizung der Hautoberfläche an dem übrigen, nicht amputierten Körper des Menschen zur Anregung von Phantomempfindungen ausführbar ist, wobei der Nachzeitraum eine Zeitdauer von weniger als 2000 ms beträgt.

In einer ergänzenden Ausgestaltung werden mehrere Komponenten des Systems stoffschlüssig und/oder kraftschlüssig und/oder formschlüssig miteinander verbunden, insbesondere wird die Steuer- und/oder Regeleinheit und/oder der Generator mit der Prothese verbunden, insbesondere mit einer Schraub- oder Klebeverbindung, und/oder das System, insbesondere wenigstens eine Komponente des Systems, dahingehend zur Verfügung gestellt oder hergestellt wird, dass das System als ein in dieser Schutzrechtsanmeldung beschriebenes System ausgebildet wird.

In einer ergänzenden Ausgestaltung werden mehrere Komponenten des Systems ohne einer mechanischen Verbindung miteinander in einer Verpackung getrennt angeordnet, insbesondere wird die Steuer- und/oder Regeleinheit getrennt von der Prothese in der Verpackung angeordnet und/oder die Steuer- und/oder Regeleinheit eine Recheneinheit, insbesondere einen Mikroprozessor, und vorzugsweise eine Datenspeichereinheit umfasst und die Recheneinheit dahingehend programmiert wird, dass das System als ein in dieser Schutzrechtsanmeldung beschriebenes System ausgebildet ist. Vorzugsweise wird das System in einer Verpackung angeordnet und/oder es erfolgt das Beilegen einer technischen Bedienungsanleitung zu dem System.

Es zeigt:
- Fig. 1: eine Ansicht eines Systems zur Anregung von Phantomempfindungen von amputierten Körpergliedern in einem ersten Ausführungsbeispiel,
- Fig. 2: eine Ansicht des Systems zur Anregung von Phantomempfindungen von amputierten Körpergliedern in einem zweiten Ausführungsbeispiel und
- Fig. 3: eine Ansicht einer Steuer- und/oder Regeleinheit des Systems.

Menschen mit einer amputierten Extremität bzw. einem amputierten Körperglied, beispielsweise einem amputierten Bein, verwenden künstliche Prothesen, z. B. eine Beinprothese 3, um die amputierte Extremität zu ersetzten und somit das tägliche Leben zu erleichtern. Die Amputation des Körpergliedes unterbricht Nervenbahnen, die im somatosensorischen Rindenfeld der gegenseitigen Hirnhälfte enden. Dies führt zu einem Zerfall von Synapsen an den Zielneuronen. Es werden neuen Synapsen gebildet, die von Nervenfasern aus benachbarten Zonen des somatosensorischen Feldes angelegt werden. Dies kann nach der Amputation beispielsweise des rechten Fußes dazu führen, dass Reizungen der Hautoberfläche 11 an dem übrigen, nicht amputierten Körper 9 des Menschen Phantomempfindungen des amputierten rechten Fußes auslöst. Es handelt sich hier um bestimmte rezeptive Hautfelder 37, 38 einer Hautoberfläche 11. Diese bestimmten rezeptive Hautfelder 37, 38 für die amputierte Extremität können beispielsweise mit taktilen Reizungen mittels Pinseln oder Bürsten auf der Hautoberfläche 11 ermittelt werden. In einem ersten Ausführungsbeispiel befinden sich die rezeptiven Hautfelder 37, 38 für den amputierten rechten Fuß an der inneren Hautoberfläche 11 der linken Hand. Die rezeptiven Hautfelder 37, 38 können bei anderen Menschen auch andere Hautoberflächen 11 betreffen, beispielsweise am Bauch oder Rücken.

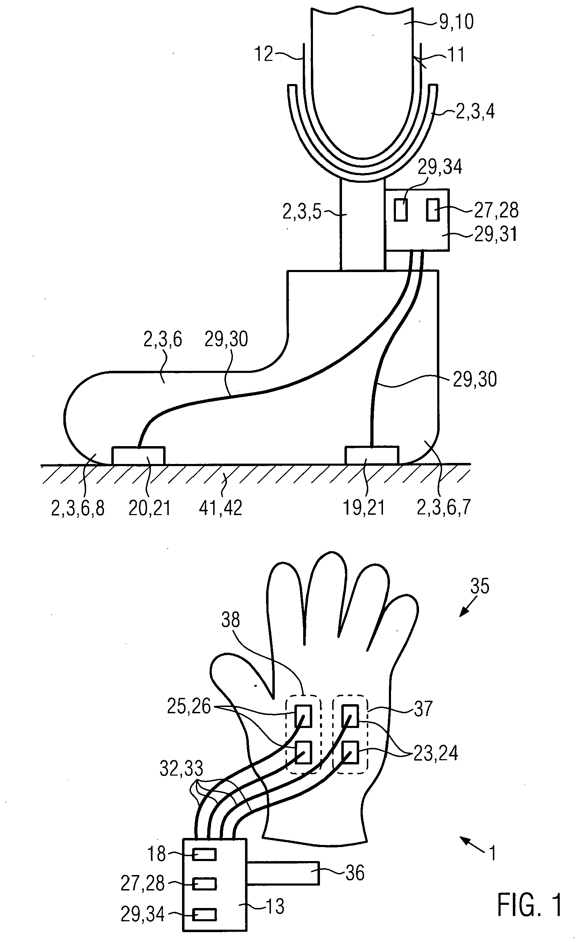

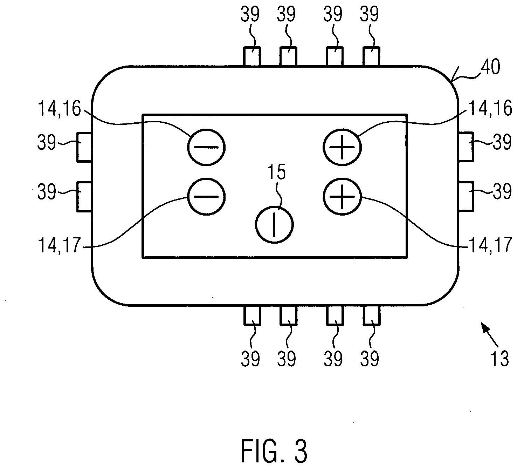

In Fig. 1 und 3 ist ein erstes Ausführungsbeispiel eines Systems 1 zur Anregung von Phantomempfindungen von amputierten Körpergliedern, nämlich eines amputierten Fußes, dargestellt. Das System 1 dient somit nicht dazu, Phantomschmerzen an amputierten Körpergliedern zu lindern, sondern Phantomempfindungen bei einem Kontakt einer Beinprothese 3 als Prothese 2 mit der Umgebung 41, d. h. einem Boden 42, anzuregen, so dass die Phantomempfindungen die haptischen Wahrnehmungen eines natürlichen Fußes annähern. Die Beinprothese 3 umfasst einen künstlichen Fuß 6 mit einer künstlichen Ferse 7 und einem künstlichen Ballen 8 am Sohlenabschnitt des Fußes 6. Der Fuß 6 ist mit einem Verbindungsstab 5 mit einem Befestigungsnapf 4 verbunden. Der Befestigungsnapf 4 dient zur Verbindung mit einem Beinstumpf 10 an dem übrigen Körper 9 des Menschen. Auf den Beinstumpf 10 ist ein Strumpf 12 bzw. ein Liner 12 aufgelegt, so dass der Beinstumpf 10 nicht unmittelbar auf dem Befestigungsnapf 4 aufliegt, sondern zwischen dem Befestigungsnapf 4 und dem Beinstumpf 10 der Liner 12 angeordnet ist. Der Liner 12 liegt somit unmittelbar an der Hautoberfläche 11 des Beinstumpfes 10 auf. In den Sohlenabschnitt des Fußes 6 an der Ferse 7 ist ein erster Sensor 19 zur Erfassung eines Kontaktes der Beinprothese 3 mit einer Umgebung 41, d. h. einem Boden 42, als ein Drucksensor 21 eingebaut und in den Ballen 8 an dem Sohlenabschnitt ist ein zweiter Sensor 20 als ein Drucksensor 21 eingebaut. Der erste und zweite Sensor 19, 20 ist jeweils mit einem Datenkabel 30 als einem drahtgebundenen Sensorverbindungsmittel 29 mit einem Verbindungsbauteil 31 verbunden. Das Verbindungsbauteil 31 mit einem Gehäuse ist lösbar mit einem nicht dargestellten Fixierungselement mit dem Verbindungsstab 5 mechanisch verbunden und das Verbindungsbauteil 31 umfasst eine Energieversorgungseinheit 27 als eine Batterie 28 und eine Antenne 34 zum Senden und/oder Empfangen von Funksignalen mit einer nicht dargestellten Sende- und/oder Empfangseinheit in dem Verbindungsbauteil 31, sodass das Verbindungsbauteil 31 auch das drahtlose Sensorverbindungsmittel 29 bildet.

Das System 1 umfasst außerdem eine Steuer- und/oder Regeleinheit 13 sowie einen Handschuh 35. Der Handschuh 35 besteht aus einem sehr dünnen, durchsichtigen Stoff und in den Handschuh 35 sind auf der Seite des Handschuhes 35, welcher auf der Innenseite der linken Hand aufliegt, eine erste Elektrode 23 und eine zweite Elektrode 25 eingebaut. Die erste Elektrode 23 ist eine erste bipolare Elektrode 24 mit einem ersten Elektrodenpaar 24 und die zweite Elektrode 25 ist eine zweite bipolare Elektrode 26 mit einem zweiten Elektrodenpaar 26. Die Elektroden 23, 25 bilden je eine Reizvorrichtung 22 zur Reizung der Hautoberfläche 11 des übrigen Körpers des Menschen. Das erste Elektrodenpaar 24 ist mit zwei getrennten Stromkabeln 33 als Reizvorrichtungsverbindungsmittel 32 mit der Steuer- und/oder Regeleinheit 13 elektrisch verbunden und analog ist das zweite Elektrodenpaar 26 mit zwei getrennten Stromkabeln 33 als drahtgebundene Reizvorrichtungsverbindungsmittel 32 mit der Steuer-und/oder Regeleinheit 13 elektrisch verbunden. Die Steuer- und/oder Regeleinheit 13 kann mit einem Befestigungsband 36 an der Hand des Menschen befestigt werden. In die Steuer- und/oder Regeleinheit 13 ist eine Energieversorgungseinheit 27 als eine Batterie 28 und eine Antenne 34 als ein drahtloses Sensorverbindungsmittel 29 eingebaut. Ein Generator 18 dient zur Erzeugung eines elektrischen Stromes mit einer in Abhängigkeit von der Zeit veränderlichen Spannung, insbesondere als Sägezahnspannung.

An einem Gehäuse 40 der Steuer- und/oder Regeleinheit 13 (Fig. 3) sind hierzu elektrische Kontaktelemente 39 ausgebildet, welche in lösbarer mechanischer und damit auch elektrischer Verbindung mit nicht dargestellten elektrischen Gegenkontaktelementen am Ende der Stromkabel 33 stehen. Nach einem Überziehen des Handschuhes 35 auf die linke Hand des Menschen liegt der Handschuh 35 an dem strichliert dargestellten ersten rezeptiven Hautfeld 37 und dem strichliert dargestellten zweiten rezeptiven Hautfeld 38 auf der Hautoberfläche 11 der linken Hand des Menschen auf. Das erste Elektrodenpaar 24 liegt ausschließlich auf dem ersten rezeptiven Hautfeld 37 auf und das zweite Elektrodenpaar 26 liegt ausschließlich auf dem zweiten rezeptiven Hautfeld 38 auf.

Während eines Gehvorganges des Menschen mit der Beinprothese 3 wird ein Kontakt zwischen der Beinprothese 3 an der Ferse 7 und dem Boden 42 mit dem ersten Sensor 19 erfasst. Mit dem Datenkabel 30 werden die von dem ersten Sensor 19 erfassten Sensordaten zu dem Verbindungsbauteil 31 übermittelt und diese Sensordaten werden anschließend in Funksignale umgewandelt und mit der Antenne 34 in dem Verbindungsbauteil 31 zu der Antenne 34 in der Steuer- und/oder Regeleinheit 13 gesendet und empfangen. In analoger Weise wird ein Kontakt zwischen der Beinprothese 3 an dem Ballen 8 und dem Boden 42 mit dem zweiten Sensor 20 erfasst. Mit dem Datenkabel 30 werden die von dem zweiten Sensor 20 erfassten Sensordaten zu dem Verbindungsbauteil 31 übermittelt und diese Sensordaten werden anschließend in Funksignale umgewandelt und mit der Antenne 34 in dem Verbindungsbauteil 31 zu der Antenne 34 in der Steuer-und/oder Regeleinheit 13 gesendet und empfangen. Die Funksignale bezüglich des ersten und zweiten Sensors 19, 20 sind unterschiedlich, so dass die Sensordaten des ersten und zweiten Sensors 19, 20 getrennt zu der Steuer- und/oder Regeleinheit 13 übersendet und empfangen werden. Der erste Sensor 19 erfasst einen Kontaktzeitraum zwischen der Ferse 7 und dem Boden 42 während des Gehens des Menschen und es tritt mit jedem Schritt des Menschen ein Kontaktzeitraum zwischen der Ferse 7 und dem Boden 42 auf. Dies gilt analog den Ballen 8. Ausschließlich simultan mit dem Kontaktzeitraum zwischen der Ferse 7 und dem Boden 42 wird aufgrund der von der Antenne 34 empfangenen Sensordaten des ersten Sensors 19 von der Steuer- und/oder Regeleinheit 13 der in dem Gehäuse 40 eingebaute Generator 40 aktiviert und nur die erste bipolare Elektrode 24 mit einem elektrischen Strom mit einer Sägezahnspannung zwischen 5 V und 10 V bei einer Frequenz von ungefähr 70 Hz als Reizsignal beaufschlagt, so dass die Spannungsdifferenz auf der Hautoberfläche 11 an dem ersten rezeptiven Hautfeld 37 eine elektrische Reizung verursacht. Analog wird ausschließlich während des Kontaktzeitraumes zwischen dem Ballen 8 und dem Boden 42 nur die zweite bipolare Elektrode 26 bestromt als Reizsignal. Die erste und zweite bipolare Elektrode 26 wird gleichzeitig bestromt, falls der Fuß 6 gleichzeitig an der Ferse 7 und dem Ballen 8 Kontakt zu der Umgebung 41 aufweist. Der Generator 18 und eine nicht dargestellte Recheneinheit, Datenspeichereinheit sowie eine nicht dargestellte Sende- und/oder Empfangseinheit 13 in der Steuer- und/oder Regeleinheit werden von der Batterie 28 mit elektrischer Energie versorgt. Der Kontaktzeitraum zwischen der Ferse 7 und dem Boden 42, welcher mit dem ersten Sensor 19 erfasst wird, ist somit im Wesentlichen identisch und simultan der Zeitdauer der Aktivität der ersten bipolaren Elektrode 24. Dies gilt analog für den zweiten Sensor 20 und die zweite bipolare Elektrode 26. Der erste Sensor 19 ist nur der ersten bipolaren Elektrode 24 zugeordnet und der zweite Sensor 20 ist nur der zweiten bipolaren Elektrode 26 zugeordnet.

Die elektrische Reizung des ersten rezeptiven Hautfeldes 37 regt Phantomempfindungen der amputierten Ferse des Menschen an und die elektrische Reizung des zweiten rezeptiven Hautfeldes 38 regt Phantomempfindungen an dem amputierten Ballen des Menschen an. Die Phantomempfinden werden im Wesentlichen zeitgleich mit der elektrischen Reizung von dem Menschen empfunden, so dass für den Menschen während des Gehens mit der Beinprothese 3 beim Auftreten mit dem Fuß 6 auf dem Boden 42 die Empfindung eines Gehens mit einem nicht amputierten Fuß künstlich angenähert erzeugt wird, weil der Mensch den Kontakt zwischen der Ferse 7 und dem Ballen 8 der Beinprothese 3 zu dem Boden 42 aufgrund der angeregten Phantomempfindungen getrennt wahrnehmen kann. Die Steuer- oder/Regeleinheit 13 weist einen Ein-AusSchalter 15 zum Ein- und Ausschalten des Systems 1 auf. Mit zwei Betätigungsorganen 14, insbesondere jeweils zwei Druckknöpfen, kann der Grad der Aktivität der Elektroden 23, 25 gesteuert werden. Mit einem ersten Schalter 16 als Betätigungsorgan 14 wird der Grad der Aktivität die erste Elektrode 23 gesteuert und mit einem zweiten Schalter 17 als Betätigungsorgan 14 wird der Grad der Aktivität der zweiten Elektrode 25 gesteuert. Der Grad der Aktivität ist umso höher, je größer die Spannungsdifferenz und/oder die Frequenz an den bipolaren Elektroden 24, 26 ist und umgekehrt. Der Mensch mit dem System 1 kann somit individuell nach seinen Bedürfnissen die Höhe der Reizung des ersten und zweiten rezeptiven Hautfeldes 37, 38 getrennt steuern.

Modern Beinprothesen 3 (nicht dargestellt) weisen wenigstens einen Elektromotor zum Bewegen von Teilen der Beinprothese 3 in Abhängigkeit von mit Sensoren 19, 20 erfassten Sensordaten auf, um den natürlichen Bewegungsablauf mit der künstlichen Beinprothese 3 zu simulieren. Der wenigstens eine Elektromotor wird von einer Steuer- und/oder Regeleinheit 13 gesteuert und/oder geregelt. Das System 1 kann in eine derartige moderne Beinprothese 3 integriert sein, indem die Sensoren 19, 20 für das System 1 mit genutzt werden und die Reizvorrichtungen 22 durch eine zusätzliche Software in der Steuer- und/oder Regeleinheit 13 gesteuert und/oder geregelt werden.

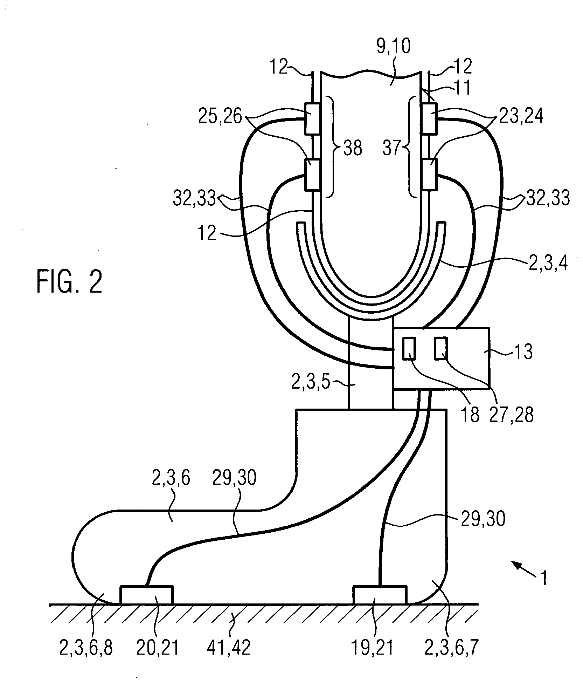

In Fig. 2 ist ein zweites Ausführungsbeispiel des Systems 1 dargestellt. Im Nachfolgenden werden im Wesentlichen nur die Unterschiede zu dem in Fig. 1 dargestellten ersten Ausführungsbeispiel beschrieben. Das System 1 weist kein Verbindungsbauteil 31 auf und die Übertragung der Sensordaten des ersten und zweiten Sensors 19, 20 zu der Steuer- und/oder Regeleinheit 13 erfolgt ausschließlich drahtgebunden mit den Datenkabeln 30, weil die Steuer- und/oder Regeleinheit 13 an dem Verbindungsstab 5 der Beinprothese 3 befestigt ist. Das erste und zweite rezeptive Hautfeld 37, 38 ist an dem Beinstumpf 10 des Menschen und die erste und zweite bipolare Elektrode 24, 26 ist in den Liner 12 bzw. Beinstrumpf 12 eingebaut bzw. integriert.

In einem weiteren, nicht dargestellten Ausführungsbeispiel ist die Prothese 2 eine Handprothese. Die Sensoren 19, 20 sind in die Handprothese, d. h. sämtliche künstlichen fünf Finger der Handprothese, eingebaut und bei einem Kontakt der Finger der Handprothese mit der Umgebung, beispielsweise einem zu greifenden Gegenstand, werden für jeden Finger der Handprothese getrennt je eine von fünf bipolaren Elektroden auf dem Rücken und Bauch des Menschen aktiviert. Die haptischen Fähigkeiten einer Person mit der Handprothese sind damit wesentlich verbessert, weil beim Greifen eines Gegenstandes mit den angeregten Phantomempfindungen das Tasten des Gegenstandes für jeden Finger als Phantomempfindung angeregt wird.

Insgesamt betrachtet sind mit dem System 1 wesentliche Vorteile verbunden. Ein Kontakt zwischen der Beinprothese 3 und der Umgebung 41 wird von den Sensoren 19, 20 getrennt an unterschiedlichen Position der Beinprothese 3 erfasst und simultan hierzu getrennt die bipolaren Elektroden 24, 26 auf den rezeptiven Hautfeldern 37, 38, mit einer Sägezahnspannung bestromt und damit ein Phantomempfinden der Umgebung angeregt. Damit werden die Empfindungen eines natürlichen Gehens ohne dem amputierten Fuß künstlich erzeugt, so dass der Mensch mit der Beinprothese 3 bessere Fähigkeiten aufweist als ohne dem System 1. Beispielsweise kann der Mensch in schwierigen Situationen besser das Gleichgewicht halten und Unebenheiten am Boden 42 aufgrund der Phantomempfindungen haptisch mittels der angeregten Phantomempfindungen erkennen

## Patentansprüche

1. System (1) zur Anregung von Phantomempfindungen von amputierten Körpergliedern, eines Menschen, umfassend:
- eine Prothese (2) zur Befestigung an dem übrigen, nicht amputierten Körper des Menschen,
- wenigstens einen in die Prothese (2) eingebauten Sensor (19, 20) zur Erfassung eines Kontaktes zwischen der Prothese (2) und der Umgebung (41) während eines Kontaktzeitraumes,
- wenigstens eine Reizvorrichtung (22) zur Reizung der Hautoberfläche (11) an dem übrigen, nicht amputierten Körper (9) des Menschen,
- eine Steuer- und/oder Regeleinheit (13) zur Steuerung und/oder Regelung der Aktivität der wenigstens einen Reizvorrichtung (22) auf der Hautoberfläche,
- eine Energieversorgungseinheit (27) zur Versorgung der Steuer-und/oder Regeleinheit (13) und der wenigstens einen Reizvorrichtung (22) mit Betriebsenergie,
- ein Sensorverbindungsmittel (29) zur Übertragung von Sensorsignalen von dem wenigstens einen Sensor (19, 20) zu der Steuer- und/oder Regeleinheit (13),
- ein Reizvorrichtungsverbindungsmittel (32) zur Übertragung von Reizsignalen von der Steuer- und/oder Regeleinheit (13) zu der wenigstens einen Reizvorrichtung (22), wobei
- die Steuer- und/oder Regeleinheit (13) dahingehend ausgebildet ist, dass während des Kontaktzeitraumes des von dem wenigstens einen Sensor (19, 20) erfassten Kontaktes zwischen der Prothese (2) und der Umgebung (41) mit der wenigstens einen Reizvorrichtung (22) eine Reizung der Hautoberfläche (11) an dem übrigen, nicht amputierten Körper (9) des Menschen ausführbar ist zur Anregung der Phantomempfindungen,
**dadurch gekennzeichnet, dass**
die Steuer- und/oder Regeleinheit (13) dahingehend ausgebildet ist, dass in einem Nachzeitraum nach dem Kontaktzeitraum mit der wenigstens einen Reizvorrichtung (22) eine Reizung der Hautoberfläche (11) an dem übrigen, nicht amputierten Körper (9) des Menschen ausführbar ist zur Anregung der Phantomempfindungen, wobei der Nachzeitraum eine Zeitdauer von weniger als 2000 ms beträgt.

2. System nach Anspruch 1
**dadurch gekennzeichnet, dass**
die Steuer- und/oder Regeleinheit (13) dahingehend ausgebildet ist, dass außerhalb des Kontaktzeitraumes und des Nachzeitraumes keine Reizung der Hautoberfläche (11) an dem übrigen, nicht amputierten Körper (9) des Menschen ausführbar ist und/oder
die Steuer- und/oder Regeleinheit (13) dahingehend ausgebildet ist, dass außerhalb des Kontaktzeitraumes und des Nachzeitraumes eine Aktivität der wenigstens einen Reizvorrichtung (22) ausgeschlossen ist.

3. System nach einem der vorhergehenden Ansprüche,
**dadurch gekennzeichnet, dass**
die wenigstens eine Reizvorrichtung (22) als je eine Elektrode (23, 25), insbesondere bipolare Elektrode (24, 26) als Elektrodenpaar (24, 26), und/oder als je eine mechanische bewegliche Reizvorrichtung ausgebildet ist
und/oder
das System (1) mehrere Reizvorrichtungen (22) zum Auflegen auf unterschiedliche Stellen der Hautoberfläche (11) umfasst, insbesondere mit der Steuer- und/oder Regeleinheit (11) die Aktivität, vorzugsweise der Grad der Aktivität, der mehreren Reizvorrichtungen (22) getrennt steuerbar und/oder regelbar ist.

4. System nach einem der vorhergehenden Ansprüche,
**dadurch gekennzeichnet, dass**
in die Prothese mehrere Sensoren (19, 20) zur Erfassung des Kontaktes zwischen der Prothese (2) und der Umgebung (41) an unterschiedlichen Positionen der Prothese (2) eingebaut sind, so dass der Kontakt der Prothese (2) zu der Umgebung (41) an unterschiedlichen Positionen der Prothese (2) getrennt erfassbar ist und/oder
der wenigstens eine Sensor (19, 20) zur Erfassung des Kontaktes zwischen der Prothese (2) und der Umgebung (41) als ein Berührungssensor und/oder ein Drucksensor (21) und/oder als ein induktiver oder kapazitiver Sensor (19, 20) ausgebildet ist.

5. System nach einem der vorhergehenden Ansprüche,
**dadurch gekennzeichnet, dass**
das System (1) einen Generator (18) zur Aktivierung der wenigstens einen Reizvorrichtung (22) umfasst, vorzugsweise der Generator (18) in die Steuer- und/oder Regeleinheit (13) eingebaut ist und/oder
die Energieversorgungseinheit (27) als eine Batterie (28), insbesondere eine aufladbare Batterie (28), vorzugsweise Lithium-Ionen-Batterie (28), ausgebildet ist
und/oder das System (1) eine Ladestation zum Aufladen der Batterie (28) umfasst
und/oder
das Sensorverbindungsmittel (29) als ein drahtloses und/oder als ein drahtgebundenes Sensorverbindungsmittel (29) ausgebildet ist und mit dem Sensorverbindungsmittel (29) Sensordaten von dem wenigstens einen Sensor (19, 20) zu der Steuer- und/oder Regeleinheit (13) und/oder umgekehrt übertragbar sind
und/oder
das Reizvorrichtungsverbindungsmittel (32) als ein drahtloses und/oder als ein drahtgebundenes Reizvorrichtungsverbindungsmittel (32) ausgebildet ist und mit dem Reizvorrichtungsverbindungsmittel (32) Reizsignale, insbesondere elektrischer Strom, von der Steuer-und/oder Regeleinheit (13) zu der wenigstens einen Reizvorrichtung (22) und/oder umgekehrt übertragbar sind
und/oder
das Sensorverbindungsmittel (29) und/oder das Reizvorrichtungsverbindungsmittel (32) und/oder die Steuer- und/oder Regeleinheit (13) und/oder die wenigstens eine Reizvorrichtung (22) und/oder der wenigstens eine Sensor (19, 20) eine Antenne (34) für Funksignale umfasst.

6. System nach einem der vorhergehenden Ansprüche,
**dadurch gekennzeichnet, dass**
mit einem Sensor (19, 20), insbesondere Drucksensor (21), ein Parameter für die zwischen einem Abschnitt der Prothese (2) mit dem Sensor (19, 20) und der Umgebung (41) wirkende Druckkraft ermittelbar ist und in Abhängigkeit von dem Parameter ein Grad der Aktivität der wenigstens einen Reizvorrichtung (22) steuerbar und/oder regelbar ist, vorzugsweise je größer der Parameter ist, desto größer der Grad der Aktivität der wenigstens einen Reizvorrichtung (22) ist und umgekehrt, insbesondere ist der Grad der Aktivität der wenigstens einen Reizvorrichtung (22) direkt proportional zu dem Parameter und vorzugsweise ist der Parameter bei einem Drucksensor (21) die zwischen einem Abschnitt der Prothese mit dem Drucksensor (21) und der Umgebung wirkenden Druckkraft selbst.

7. System nach einem der vorhergehenden Ansprüche,
**dadurch gekennzeichnet, dass**
die wenigstens eine Reizvorrichtung (22) in einen Handschuh (35) und/oder in einen Liner bzw. Strumpf (12) eingebaut ist und/oder
die Prothese (2) als eine Beinprothese (3) oder eine Handprothese oder eine Brustprothese ausgebildet ist, vorzugsweise die Sensoren (19, 20) an unterschiedlichen Positionen im Sohlenabschnitt der Beinprothese (3) eingebaut sind oder die Sensoren (19, 20) in unterschiedlichen künstlichen Fingern der Handprothese eingebaut sind.

8. Verfahren zur Herstellung eines Systems (1) zur Anregung von Phantomempfindungen von amputierten Körpergliedern eines Menschen, insbesondere eines Systems (1) nach einem oder mehreren der vorhergehenden Ansprüche, mit Schritten:
- zur Verfügung stellen einer Prothese (2) als Komponente des Systems (1) zur Befestigung an dem übrigen, nicht amputierten Körper (9) des Menschen,
- zur Verfügung stellen von wenigstens einem Sensor (19, 20) als Komponente des Systems (1) zur Erfassung eines Kontaktes zwischen der Prothese (2) und der Umgebung (41) während eines Kontaktzeitraumes,
- zur Verfügung stellen wenigstens einer Reizvorrichtung (22) als Komponente des Systems (1) zur Reizung der Hautoberfläche (11) an dem übrigen, nicht amputierten Körper (9) des Menschen,
- zur Verfügung steilen einer Steuer- und/oder Regeleinheit (13) als Komponente des Systems (1) zur Steuerung und/oder Regelung der Aktivität der wenigstens einen Reizvorrichtung (22) auf der Hautoberfläche (11),
- zur Verfügung stellen einer Energieversorgungseinheit (27) als Komponente des Systems (1) zur Versorgung der Steuer-und/oder Regeleinheit (13) und der wenigstens einen Reizvorrichtung (22) mit Betriebsenergie,
- zur Verfügung stellen eines Sensorverbindungsmittels (29) als Komponente des Systems (1) zur Übertragung von Sensorsignalen von dem wenigstens einen Sensor (19, 20) zu der Steuer- und/oder Regeleinheit (13),
- zur Verfügung stellen eines Reizvorrichtungsverbindungsmittel (32) als Komponente des Systems (1) zur Übertragung von Reizsignalen von der Steuer- und/oder Regeleinheit (13) zu der wenigstens einen Reizvorrichtung (22),
- vorzugsweise zur Verfügung stellen eines Generators (18) als Komponente des Systems (1) zur Aktivierung der wenigstens einen Reizvorrichtung (22), wobei
- die Steuer- und/oder Regeleinheit (13) dahingehend zur Verfügung gestellt wird, dass während des Kontaktzeitraumes des von dem wenigstens einen Sensor (19, 20) erfassten Kontaktes zwischen der Prothese (2) und der Umgebung (41) mit der wenigstens einen Reizvorrichtung (22) eine Reizung der Hautoberfläche (11) an dem übrigen, nicht amputierten Körper (9) des Menschen zur Anregung der Phantomempfindungen ausführbar ist,
**dadurch gekennzeichnet, dass**
die Steuer- und/oder Regeleinheit (13) dahingehend zur Verfügung gestellt wird, dass in einem Nachzeitraum nach dem Kontaktzeitraum mit der wenigstens einen Reizvorrichtung (22) eine Reizung der Hautoberfläche (11) an dem übrigen, nicht amputierten Körper (9) des Menschen zur Anregung der Phantomempfindungen ausführbar ist, wobei der Nachzeitraum eine Zeitdauer von weniger als 2000 ms beträgt.

9. Verfahren nach Anspruch 8,
**dadurch gekennzeichnet, dass**
mehrere Komponenten (2, 13, 18, 19, 20, 22, 27, 29, 32) des Systems (1) stoffschlüssig und/oder kraftschlüssig und/oder formschlüssig miteinander verbunden werden, insbesondere die Steuer- und/oder Regeleinheit (13) und/oder der Generator (18) mit der Prothese (2) verbunden wird,
und/oder
das System (1), insbesondere wenigstens eine Komponente (2, 13, 18, 19, 20, 22, 27, 29, 32) des Systems (1), dahingehend zur Verfügung gestellt oder hergestellt wird, dass das System (1) gemäß einem oder mehrerer der Ansprüche 1 bis 7 ausgebildet wird.

10. Verfahren nach Anspruch 8 oder 9,
**dadurch gekennzeichnet, dass**
mehrere Komponenten des Systems (1) ohne einer mechanischen Verbindung miteinander in einer Verpackung getrennt angeordnet werden, insbesondere die Steuer- und/oder Regeleinheit (1) getrennt von der Prothese (2) in der Verpackung angeordnet wird und/oder
die Steuer- und/oder Regeleinheit (13) eine Recheneinheit, insbesondere einen Mikroprozessor, und vorzugsweise eine Datenspeichereinheit umfasst und die Recheneinheit dahingehend programmiert wird, dass das System (1) gemäß einem oder mehreren der Ansprüche 1 bis 7 ausgebildet ist.

## Claims

1. System (1) for stimulating phantom sensations of amputated limbs of a person, comprising:
- a prosthesis (2) for attachment to the remaining, non-amputated body of the person,
- at least one in the prosthesis (2) built-in sensor (19, 20) for detecting a contact between the prosthesis (2) and the surrounding (41) during a contact time period,
- at least one stimulation device (22) for stimulating the skin surface (11) on the remaining, non-amputated body (9) of the person,
- a control and/or regulating unit (13) for controlling and/or regulating the activity of the at least one stimulation device (22) on the skin surface (11),
- a power supply unit (27) for supplying the control and/or regulating unit (13) and the at least one stimulation device (22) with operating power,
- a sensor connection means (29) for transmitting sensor signals from the at least one sensor (19, 20) to the control and/or regulating unit (13),
- a stimulation device connection means (32) for transmitting stimulation signals from the control and/or regulating unit (13) to the at least one stimulation device (22), whereby
- the control and/or regulating unit (13) being designed such that, during the contact time period detected by the at least one sensor (19, 20) between the prosthesis (2) and the surrounding (41) a stimulation of the skin surface (11) on the remaining, non-amputated body (9) of the person is executable for the stimulation of phantom sensations,
**characterized in that**
the control and/or regulating unit (13) being designed such that, in a subsequent time period after the contact time period a stimulation of the skin surface (11) on the remaining, non-amputated body (9) of the person is executable for the stimulation of phantom sensations, whereby subsequent time period is a time period of less than 2000 ms.

2. System according to claim 1,
**characterized in that**
the control and/or regulating unit (13) being designed that, beyond of the contact time period and the subsequent time period no stimulation of the skin surface (11) is executable on the remaining, non-amputated body (9) of the person
and/or
the control and/or regulating unit (13) being designed that, beyond the contact time period and the subsequent time period the activity of at least one stimulation device (22) is excluded.

3. System according to one of the preceding claims,
**characterized in that**
the at least one stimulation device (22) is constructed each as an electrode (23, 25), in particular bipolar electrode (24, 26) as a pair of electrodes (24, 26), and/or each as a mechanical stimulation movable device
and/or
the system (1) comprises a plurality of stimulation devices (22) to be placed on different parts of the skin surface (11), in particular with the control and/or regulating unit (11) the activity, preferably the level of activity, of several stimulation devices (22) is separately controlled and/or regulated.

4. System according to one of the preceding claims,
**characterized in that**
in the prosthesis several sensors (19, 20) for detecting the contact between the prosthesis (2) and the surrounding (41) at different positions of the prosthesis (2) are installed, so that the contact of the prosthesis (2) to the surrounding (41) at different positions of the prosthesis (2) is separately sensed
and/or
at least one sensor (19, 20) for detecting the contact between the prosthesis (2) and the surrounding (41) is constructed as a touch sensor and/or a pressure sensor (21) and/or as an inductive or capacitive sensor (19, 20).

5. System according to one of the preceding claims,
**characterized in that**
the system (1) comprises a generator (18) for activating the at least one stimulation device (22), preferably the generator (18) is installed in the control and/or regulating unit (13)
and/or
the power supply unit (27) is constructed as a battery (28), in particular a rechargeable battery (28), preferably lithium-ion battery (28)
and/or
the system (1) comprises a charging station for charging the battery (28)
and/or
the sensor connection means (29) is constructed as a wireless and/or as a wired sensor connection means (29) and with the sensor connection means (29) sensor data from the at least one sensor (19, 20) is transferable to the control and/or regulating unit (13) and/or vice versa
and/or
the stimulation device connection means (32) is constructed as a wireless and/or as a wired stimulation device connection means (32) and with the stimulation device connection means (32) stimulation signals, in particular electric current, are transferable form the control and/or regulating unit (13) to the at least one stimulation device (22) and/or vice versa
and/or
the sensor connection means (29) and/or the stimulation device connection means (32) and/or the control and/or regulating unit (13) and/or the at least one stimulation device (22) and/or the at least one sensor (19, 20) comprises an antenna (34) for radio signals.

6. System according to one of the preceding claims,
**characterized in that**
with a sensor (19, 20), in particular pressure sensor (21), a parameter for the pressure force between a part of the prosthesis (2) with the sensor (19, 20) and the surrounding (41) is ascertainable and depending on the parameter a level of activity of the at least one stimulation device (22) is controlled and/or regulated, preferably the larger the parameter is, the greater is the degree of activity of the at least one stimulation device (22) and vice versa, in particular the degree of activity of the at least one stimulation device (22) is directly proportional to the parameter and preferably the parameter is for a pressure sensor (21) the pressure force itself between a part of the prosthesis (2) with the pressure sensor (21) and the surrounding.

7. System according to one of the preceding claims,
**characterized in that**
the at least one stimulation device (22) is installed in a glove (35) and/or in a liner (12) respectively sock
and/or
the prosthesis (2) is constructed as a leg prosthesis (3) or a hand prosthesis or a breast prosthesis, preferably the sensors (19, 20) are installed at different positions in the sole portion of the leg prosthesis (3) or the sensors (19, 20) are installed in different artificial fingers of the hand prosthesis.

8. A process for the production of system (1) for stimulating phantom sensations of amputated limbs of a person, in particular a system (1) according to one or more of the preceding claims, with the steps:
- provide a prosthesis (2) as a component of the system (1) for attachment to the remaining, non-amputated body (9) of the person
- provide at least one sensor (19, 20) as a component of the system (1) for detecting a contact between the prosthesis (2) and the surrounding (41) during a contact time period,
- provide at least one stimulation device (22) as a component of the system (1) for stimulating the skin surface (11) on the remaining, not amputated body (9) of the person,
- provide a control and/or regulating unit (13) as a component of the system (1) for controlling and/or regulating the activity of the at least one stimulation device (22) on the skin surface (11),
- provide a power supply unit (27) as a component of the system (1) for supplying the control and/or regulating unit (13) and the at least one stimulation device (22) with operating power,
- provide a sensor connection means (29) as a component of the system (1) for transmitting sensor signals from the at least one sensor (19, 20) to the control and/or regulating unit (13),
- provide a stimulation device connection means (32) as a component of the system (1) for transmitting stimulation signals from the control and/or regulating unit (13) to the at least one stimulation device (22),
- preferably provide a generator (18) as a component of the system (1) for activating the at least one stimulation device (22),
- the control and/or regulating unit (13) is provided to the effect that, during the contact time period detected by the at least one sensor (19, 20) between the prosthesis (2) and the surrounding (41) a stimulation of the skin surface (11) on the remaining, non-amputated body (9) of the person is executable for the stimulation of phantom sensations,
**characterized in that**
the control and/or regulating unit (13) is provided to the effect that, in a subsequent time period after the contact time period a stimulation of the skin surface (11) on the remaining, non-amputated body (9) of the person is executable for the stimulation of phantom sensations, whereby subsequent time period is a time period of less than 2000 ms.

9. A method according to claim 8,
**characterized in that**
a plurality of components (2, 13, 18, 19, 20, 22, 27, 29, 32) of the system (1) are connected to one another by material-locking and/or force-locking and/or form-locking, in particular the control and/or regulating unit (13) and/or the generator (18) is connected with the prosthesis (2)
and/or
the system (1), in particular at least one component (2, 13, 18, 19, 20, 22, 27, 29, 32) of the system (1), is provided or produced in a way that the system (1) is constructed according to one or more of claims 1 to 7.

10. The method according to claim 8 or 9,
**characterized in that**
several components of the system (1) are separately arranged without a mechanical connection with each other in a package, in particular the control and/or regulating unit (1) is separately arranged from the prosthesis (2) in the package
and/or
the control and/or regulating unit (13) comprises a computing unit, in particular a microprocessor, and preferably a data storage unit and the computing unit is programmed in a way that the system (1) is constructed according to one or more of claims 1 to 7.

## Revendications

1. Système (1) d'excitation de sensations fantômes provenant des membres amputés d'une personne, comprenant :
- une prothèse (2) à fixer au reste du corps, non amputé, de la personne,
- au moins un capteur (19, 20) installé dans la prothèse (2) pour détecter un contact entre la prothèse (2) et l'environnement (41) pendant une période de contact,
- au moins un dispositif de stimulation (22) pour stimuler la surface de la peau (11) sur le reste du corps (9), non amputé, de la personne,
- une unité de commande et/ou de régulation (13) pour commander et/ou réguler l'activité dudit au moins un dispositif de stimulation (22) à la surface de la peau,
- une unité d'alimentation en énergie (27) pour alimenter l'unité de commande et/ou de régulation (13) et ledit au moins un dispositif de stimulation (22) en énergie de fonctionnement,
- un moyen de connexion de capteur (29) pour transmettre des signaux de capteur dudit au moins un capteur (19, 20) à l'unité de commande et/ou de régulation (13),
- un moyen de connexion de dispositif de stimulation (32) pour transmettre des signaux de stimulation de l'unité de commande et/ou de régulation (13) audit au moins un dispositif de stimulation (22), dans lequel
- l'unité de commande et/ou de régulation (13) est réalisée de telle sorte que pendant la période de contact du contact entre la prothèse (2) et l'environnement (41), détecté par ledit au moins un capteur (19, 20), ledit au moins un dispositif de stimulation (22) permet d'effectuer une stimulation de la surface de la peau (11) sur le reste du corps (9), non amputé, de la personne afin d'exciter les sensations fantômes,
**caractérisé en ce que**
l'unité de commande et/ou de régulation (13) est réalisée de telle sorte que dans une période postérieure, après la période de contact avec ledit au moins un dispositif de stimulation (22), ledit au moins un dispositif de stimulation (22) permet d'effectuer une stimulation de la surface de la peau (11) sur le reste du corps (9), non amputé, de la personne afin d'exciter les sensations fantômes, la période postérieure étant inférieure à 2000 ms.

2. Système selon la revendication 1, **caractérisé en ce que**
l'unité de commande et/ou de régulation (13) est réalisée de telle sorte qu'en dehors de la période de contact et de la période postérieure, aucune stimulation de la surface de la peau (11) ne peut être effectuée sur le reste du corps (9), non amputé, de la personne,
et/ou
l'unité de commande et/ou de régulation (13) est réalisée de telle sorte qu'en dehors de la période de contact et de la période postérieure, toute activité dudit au moins un dispositif de stimulation (22) est exclue.

3. Système selon l'une quelconque des revendications précédentes, **caractérisé en ce que**
ledit au moins un dispositif de stimulation (22) est réalisé comme une électrode (23, 25) respectivement, en particulier une électrode bipolaire (24, 26), comme une paire d'électrodes (24, 26) et/ou comme un dispositif de stimulation mobile mécanique respectivement,
et/ou
le système (1) comprend plusieurs dispositifs de stimulation (22) à poser à différents endroits de la surface de la peau (11), en particulier l'unité de commande et/ou de régulation (11) permettant de commander et/ou de réguler séparément l'activité, de préférence le degré d'activité, des plusieurs dispositifs de stimulation (22).

4. Système selon l'une quelconque des revendications précédentes, **caractérisé en ce que**
dans la prothèse, plusieurs capteurs (19, 20) pour détecter le contact entre la prothèse (2) et l'environnement (41) sont installés à différentes positions de la prothèse (2) de sorte que le contact de la prothèse (2) avec l'environnement (41) peut être détecté séparément à différentes positions de la prothèse (2),
et/ou
ledit au moins un capteur (19, 20) pour détecter le contact entre la prothèse (2) et l'environnement (41) est réalisé comme un capteur de contact et/ou un capteur de pression (21) et/ou comme un capteur inductif ou capacitif (19, 20).

5. Système selon l'une quelconque des revendications précédentes, **caractérisé en ce que**
le système (1) comprend un générateur (18) pour activer ledit au moins un dispositif de stimulation (22), le générateur (18) étant de préférence intégré dans l'unité de commande et/ou de régulation (13),
et/ou
l'unité d'alimentation en énergie (27) est réalisée comme une batterie (28), en particulier une batterie rechargeable (28), de préférence une batterie lithium-ion (28),
et/ou
le système (1) comprend une station de charge pour recharger la batterie (28),
et/ou
le moyen de connexion de capteur (29) est réalisé comme un moyen de connexion de capteur (29) sans fil et/ou filaire, et le moyen de connexion de capteur (29) permettant de transmettre des données de capteur dudit au moins un capteur (19, 20) à l'unité de commande et/ou de régulation (13) et/ou inversement,
et/ou
le moyen de connexion de dispositif de stimulation (32) est réalisé comme un moyen de connexion de dispositif de stimulation (32) sans fil et/ou filaire, et le moyen de connexion de dispositif de stimulation (32) permettant de transmettre des signaux de stimulation, en particulier du courant électrique, de l'unité de commande et/ou de régulation (13) audit au moins un dispositif de stimulation (22) et/ou inversement,
et/ou
le moyen de connexion de capteur (29) et/ou le moyen de connexion de dispositif de stimulation (32) et/ou l'unité de commande et/ou de régulation (13) et/ou ledit au moins un dispositif de stimulation (22) et/ou ledit au moins un capteur (19, 20) comprennent une antenne (34) pour des signaux radio.

6. Système selon l'une quelconque des revendications précédentes, **caractérisé en ce que** le capteur (19, 20), en particulier le capteur de pression (21), permet de déterminer un paramètre pour la force de pression agissant entre une partie de la prothèse (2) munie du capteur (19, 20) et l'environnement (41), et en fonction du paramètre, un degré de l'activité dudit au moins un dispositif de stimulation (22) peut être commandé et/ou régulé, de préférence plus le paramètre est grand, plus le degré de l'activité dudit au moins un dispositif de stimulation (22) est grand et inversement, en particulier le degré de l'activité dudit au moins un dispositif de stimulation (22) étant directement proportionnel au paramètre, et de préférence pour un capteur de pression (21), le paramètre étant la force de pression elle-même qui agit entre une partie de la prothèse munie du capteur de pression (21) et l'environnement.

7. Système selon l'une quelconque des revendications précédentes, **caractérisé en ce que**
ledit au moins un dispositif de stimulation (22) est installé dans un gant (35) et/ou dans une doublure ou une chaussette (12),
et/ou
la prothèse (2) est réalisée comme une prothèse de jambe (3) ou une prothèse de main ou une prothèse mammaire, de préférence les capteurs (19, 20) étant installés à différentes positions dans la partie de semelle de la prothèse de jambe (3) ou les capteurs (19, 20) étant installés dans différents doigts artificiels de la prothèse de main.

8. Procédé de fabrication d'un système (1) d'excitation de sensations fantômes des membres amputés d'une personne, en particulier d'un système (1) selon une ou plusieurs des revendications précédentes, comprenant les étapes consistant à :
- prévoir une prothèse (2) comme un composant du système (1) à fixer sur le reste du corps (9), non amputé, de la personne,
- prévoir au moins un capteur (19, 20) comme un composant du système (1) pour détecter un contact entre la prothèse (2) et l'environnement (41) pendant une période de contact,
- prévoir au moins un dispositif de stimulation (22) comme un composant du système (1) pour stimuler la surface de la peau (11) sur le reste du corps (9), non amputé, de la personne,
- prévoir une unité de commande et/ou de régulation (13) comme un composant du système (1) pour commander et/ou réguler l'activité dudit au moins un dispositif de stimulation (22) à la surface de la peau (11),
- prévoir une unité d'alimentation en énergie (27) comme un composant du système (1) pour alimenter l'unité de commande et/ou de régulation (13) et ledit au moins un dispositif de stimulation (22) en énergie de fonctionnement,
- prévoir un moyen de connexion de capteur (29) comme un composant du système (1) pour transmettre des signaux de capteur dudit au moins un capteur (19, 20) à l'unité de commande et/ou de régulation (13),
- prévoir un moyen de connexion de dispositif de stimulation (32) comme un composant du système (1) pour transmettre des signaux de stimulation de l'unité de commande et/ou de régulation (13) audit au moins un dispositif de stimulation (22),
- de préférence, prévoir un générateur (18) comme un composant du système (1) pour activer ledit au moins un dispositif de stimulation (22), dans lequel
- l'unité de commande et/ou de régulation (13) est prévue de telle sorte que pendant la période de contact du contact entre la prothèse (2) et l'environnement (41), détecté par ledit au moins un capteur (19, 20), ledit au moins un dispositif de stimulation (22) permet d'effectuer une stimulation de la surface de la peau (11) sur le reste du corps (9), non amputé, de la personne afin d'exciter les sensations fantômes,
**caractérisé en ce que**
l'unité de commande et/ou de régulation (13) est prévue de telle sorte que dans une période postérieure, après la période de contact avec ledit au moins un dispositif de stimulation (22), une stimulation de la surface de la peau (11) peut être effectuée sur le reste du corps (9), non amputé, de la personne afin d'exciter les sensations fantômes, la période postérieure étant inférieure à 2000 ms.

9. Procédé selon la revendication 8, **caractérisé en ce que**
plusieurs composants (2, 13, 18, 19, 20, 22, 27, 29, 32) du système (1) sont reliés les uns aux autres par liaison de matière et/ou par adhérence et/ou par complémentarité de forme, en particulier l'unité de commande et/ou de régulation (13) et/ou le générateur (18) étant reliés à la prothèse (2),
et/ou
le système (1), en particulier au moins un composant (2, 13, 18, 19, 20, 22, 27, 29, 32) du système (1), est prévu de telle sorte que le système (1) est réalisé selon une ou plusieurs des revendications 1 à 7.

10. Procédé selon la revendication 8 ou 9, **caractérisé en ce que** plusieurs composants du système (1) sont disposés séparément dans un emballage, sans connexion mécanique les uns avec les autres, en particulier l'unité de commande et/ou de régulation (1) étant disposée dans l'emballage séparément de la prothèse (2),
et/ou
l'unité de commande et/ou de régulation (13) comprend une unité de calcul, en particulier un microprocesseur, et de préférence une unité de stockage de données, et l'unité de calcul est programmée de telle sorte que le système (1) est réalisé selon une ou plusieurs des revendications 1 à 7.
